# EUROPEAN PATENT SPECIFICATION

(11) **EP 1 904 478 B1**
(45) Date of publication and mention of the grant of the patent: **24.07.2013**
(21) Application number: 06741240.3
(22) Date of filing: 15.06.2006
(51) Int. Cl.: C07D 401/12, A61K 31/506, A61P 17/06, A61P 31/04, A61P 31/12, A61P 35/00, A61P 37/00

(54) **ACID ADDITION SALTS OF N-ETHYL-N'-[2-METHOXY-4-(5-METHYL-4-{[(1S)-1-PYRIDIN-3-YLBUTYL]AMINO}PYRIMIDIN- 2-YL)PHENYL]UREA AND USES THEREOF**
SÄUREADDITIONSSALZE VON N-ETHYL-Ný-[2-METHOXY-4-(5-METHYL-4-{[(S)-1-PYRIDIN-3-YLBUTYL]AMINO}PYRIMIDIN-2-YL)PHENYL]HARNSTOFF UND ANWENDUNGEN DAVON
SELS D'ADDITION D'ACIDE DE LA N-ÉTHYL-N -[2-MÉTHOXY-4-(5-MÉTHYL-4-{[(1S)-1-PYRIDIN-3-YLBUTYL]AMINO}PYRIMIDIN-2-YL)PHÉNYL]URÉE ET LEURS UTILISATIONS

(30) Priority: 15.06.2005 AU 2005903101; 15.06.2005 AU 2005903104; 15.06.2005 AU 2005903105; 15.06.2005 AU 2005903103; 15.06.2005 AU 2005903102
(43) Date of publication of application: 02.04.2008
(73) Proprietor: YM BioSciences Australia Pty Ltd, Melbourne VIC 3004 (AU)
(72) Inventor: BURNS, Christopher John, North Caulfield, Victoria 3161 (AU); HARTE, Michael Francis, Viewbank, Victoria 3084 (AU)
(74) Representative: Lenthall, Joseph
(86) International application number: PCT/AU2006/000829
(87) International publication number: WO 2006/133498

(56) References cited:
- WO-A1-2004/052868
- WO-A1-2005/054199

## Description

### FIELD OF THE INVENTION

The invention relates to formation of acid addition salts of N-ethyl-N'-[2-methoxy-4-(5- methyl-4-{[(1S)-1-pyridin-3-ylbutyl]amino}pyrimidin-2-yl)phenyl]urea (Formula I), pharmaceutical compositions containing those addition salts, and the salts for use in the therapeutic treatment of warm-blooded animals, especially humans, or their use for the preparation of pharmaceutical preparations for use in the therapeutic treatment of warm-blooded animals, especially humans.

### BACKGROUND OF THE INVENTION

The preparation of N-ethyl-N'-[2-methoxy-4-(5-methyl-4-{[(1S)-1-pyridin-3-ylbutyl] amino}pyrimidin-2-yl)phenyl]urea and the use thereof, especially as an anti-tumour agent, are described in our co-pending International Patent Application No. PCT/AU2004/001689 (WO 2005/054199). This compound is exemplified in this publication only as its free base form (not as a salt).

WO 2004/052868 describes pyrazine derivatives for the treatment of hyperproliferative diseases.

It has now been surprisingly found that acid addition salts of this compound have advantageous properties.

### SUMMARY OF THE INVENTION

Accordingly, in a first aspect, the present invention provides an acid addition salt of a compound of Formula I: 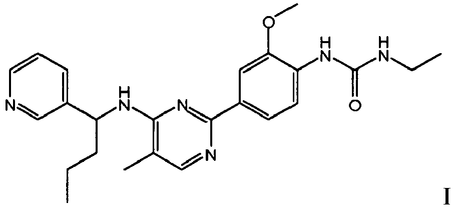 wherein the acid is selected from the group consisting of citric acid, hydrochloric acid, methanesulfonic acid, oxalic acid and tartaric acid.

In a second aspect, the present invention provides an acid addition salt according to the first aspect for use in a method of treating a hyperproliferation-related disorder.

In a third aspect, the present invention provides the use of an acid addition salt according to the first aspect in the preparation of a medicament for the treatment of a hyperproliferation-related disorder.

In a fourth aspect, the present invention provides a pharmaceutical composition comprising an acid addition salt according to the first aspect and a pharmaceutically acceptable carrier.

### BRIEF DESCRIPTION OF THE FIGURES

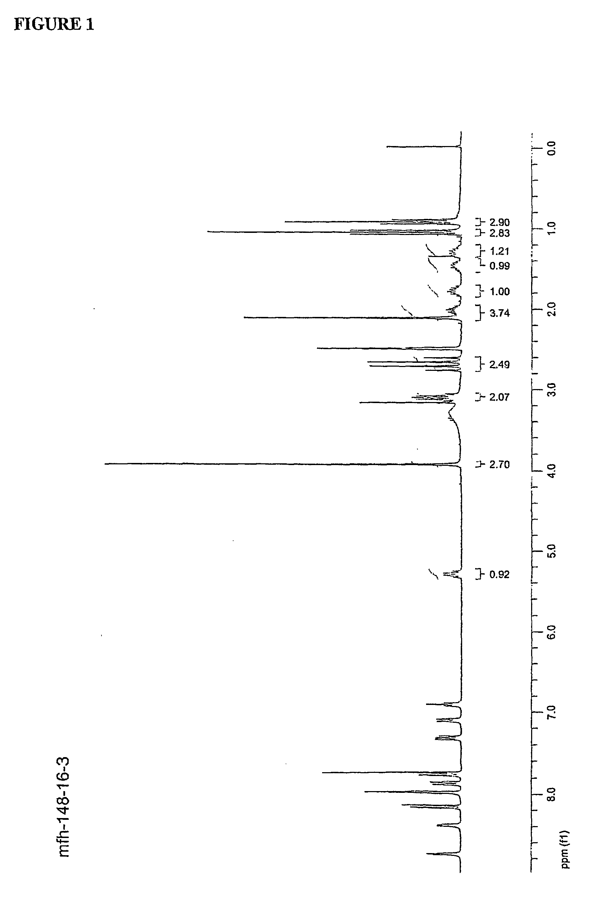

**Figure 1** shows the ¹H-NMR spectrum of a citric acid addition salt of the compound of formula I. The 300-MHz ¹H-NMR spectra was obtained at 300 K using 15 mg of the citric acid addition salt of N-ethyl-N'-[2-methoxy-4-(5-methyl-4-{[(1S)-1-pyridin-3-ylbutyl]amino}pyrimidin-2-yl)phenyl]urea dissolved in 0.5 mL of DMSO-*d*₆.

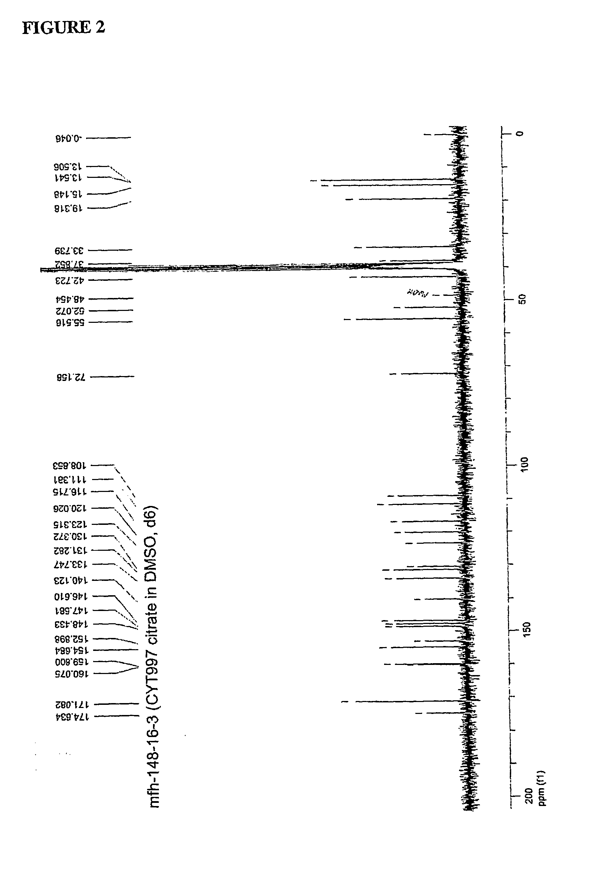

**Figure 2** shows the ¹³C-NMR spectrum of a citric acid addition salt of the compound of formula I. The 125-MHz ¹³C-NMR spectra were obtained at 300 K using 80 mg of the citric acid addition salt ofN-ethyl-N'-[2-methoxy-4-(5-methyl-4- {[(1S)-1-pyridin-3-ylbutyl]amino}pyrimidin-2-yl)phenyl]urea dissolved in 0.5 mL of DMSO-*d*₆.

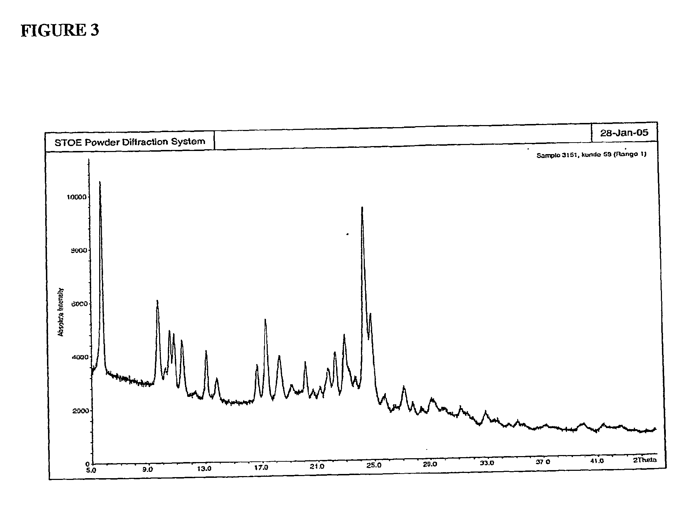

**Figure 3** shows the X-ray powder diffraction pattern of the crystal form of a hydrochloric acid addition salt of the compound of formula 1. The powder X-ray diffraction (XRD) pattern indicates that the hydrochloric acid addition salt of N-ethyl-N'-[2-methoxy-4-(5-methyl-4-{[(1S)-1-pyridin-3-ylbutyl]amino}pyrimidin-2-yl)phenyl]urea has a medium to high degree of crystallinity.

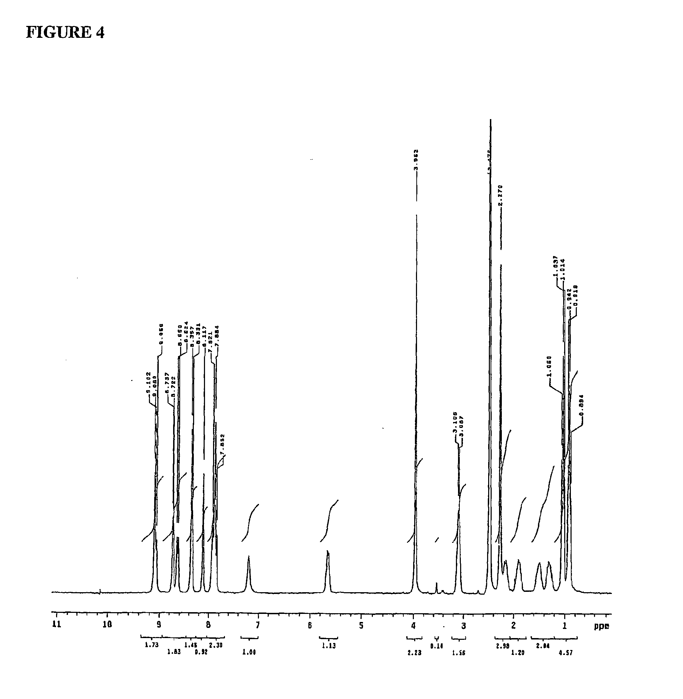

**Figure 4** shows the ¹H-NMR spectrum of a hydrochloric acid addition salt of the compound of formula I. The 300-MHz ¹H-NMR spectrum was obtained at 300 K using 15 mg of the hydrochloric acid addition salt of N-ethyl-N'-[2-methoxy-4-(5-methyl-4-{[(1S)-1-pyridin-3-ylbutyl]amino}pyrimidin-2-yl)phenyl]urea dissolved in 0.5 mL of DMSO-*d₆*.

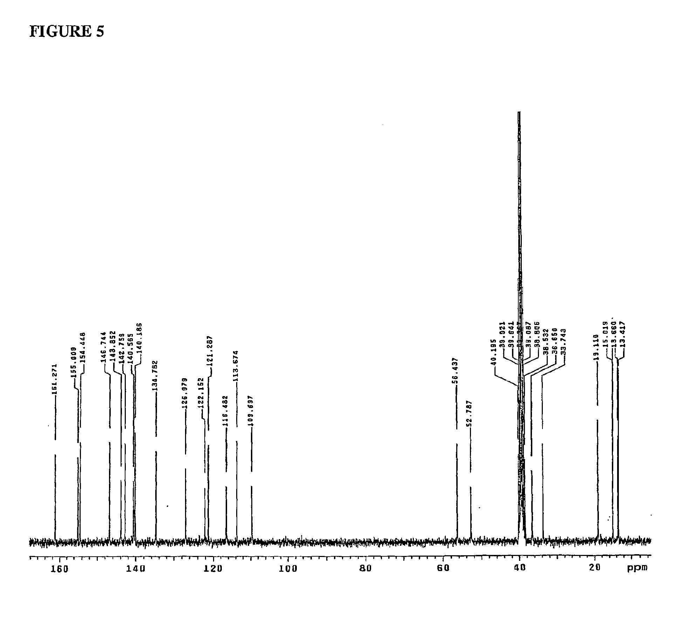

**Figure 5** shows the ¹³C-NMR spectrum of a hydrochloric acid addition salt of the compound of formula I. The 125-MHz ¹³C-NMR spectrum was obtained at 300 K using 80 mg of the hydrochloric acid addition salt of N-ethyl-N'-[2-methoxy-4-(5-methyl-4-{[(1S)-1-pyridin-3-ylbutyl]amino}pyrimidin-2-yl)phenyl]urea dissolved in 0.5 mL of DMSO-*d₆*.

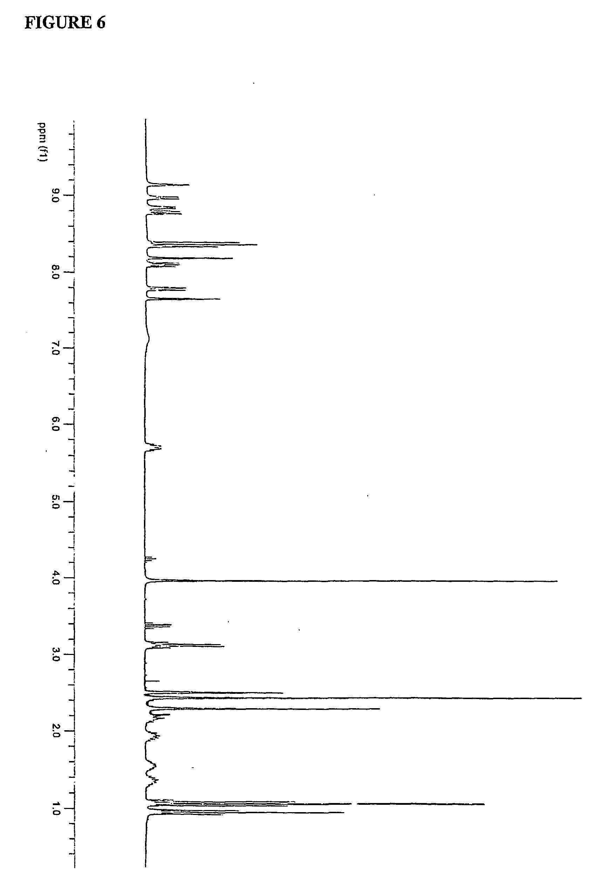

**Figure 6** shows the ¹H-NMR spectrum of a methanesulfonic acid addition salt of the compound of formula I. The 300-MHz ¹H-NMR spectra was obtained at 300 K using 20 mg of the methanesulfonic acid addition salt of N-ethyl-N'-[2-methoxy-4-(5-methyl-4-{[(1S)-1-pyridin-3-ylbutyl]amino}pyrimidin-2-yl)phenyl]urea dissolved in 0.5 mL of DMSO-*d₆*.

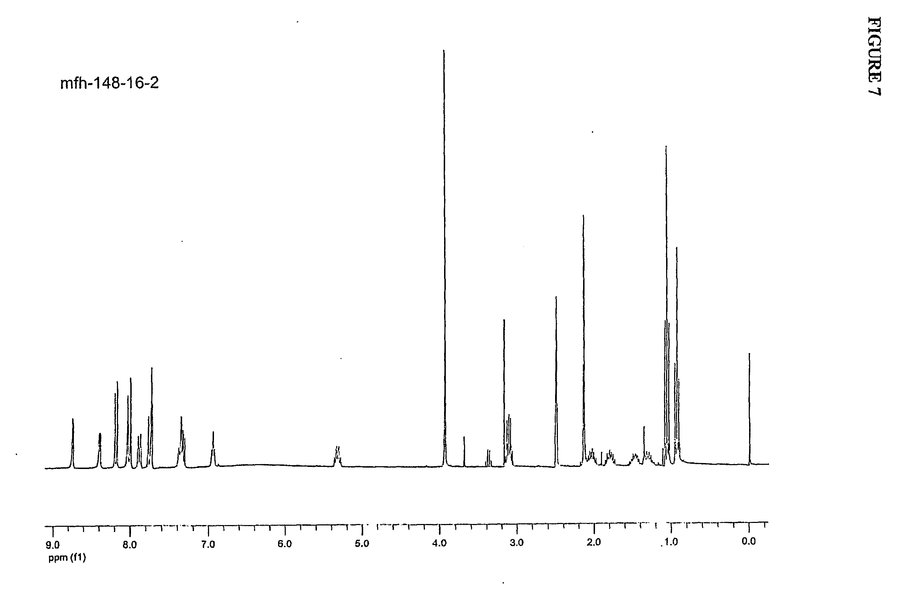

**Figure 7** shows the ¹H-NMR spectrum of an oxalic acid addition salt of the compound of formula I. The 300-MHz ¹H-NMR spectra was obtained at 300 K using 15 mg of the oxalic acid addition salt of N-ethyl-N'-[2-methoxy-4-(5-methyl-4-{[(1S)-1-pyridin-3-ylbutyl]amino}pyrimidin-2-yl)phenyl]urea dissolved in 0.5 mL of DMSO-*d₆*.

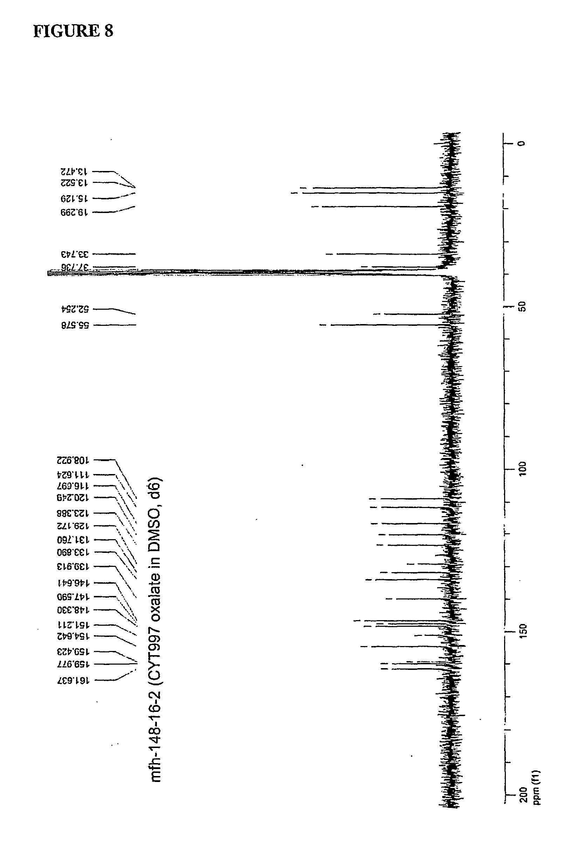

**Figure 8** shows the ¹³C-NMR spectrum of an oxalic acid addition salt of the compound of formula 1. The 125-MHz ¹³C-NMR spectra were obtained at 300 K using 80 mg of the oxalic acid addition salt of N-ethyl-N'-[2-methoxy-4-(5-methyl-4-{[(1S)-1-pyridin-3-ylbutyl]amino}pyrimidin-2-yl)phenyl]urea dissolved in 0.5 mL of DMSO-*d₆*.

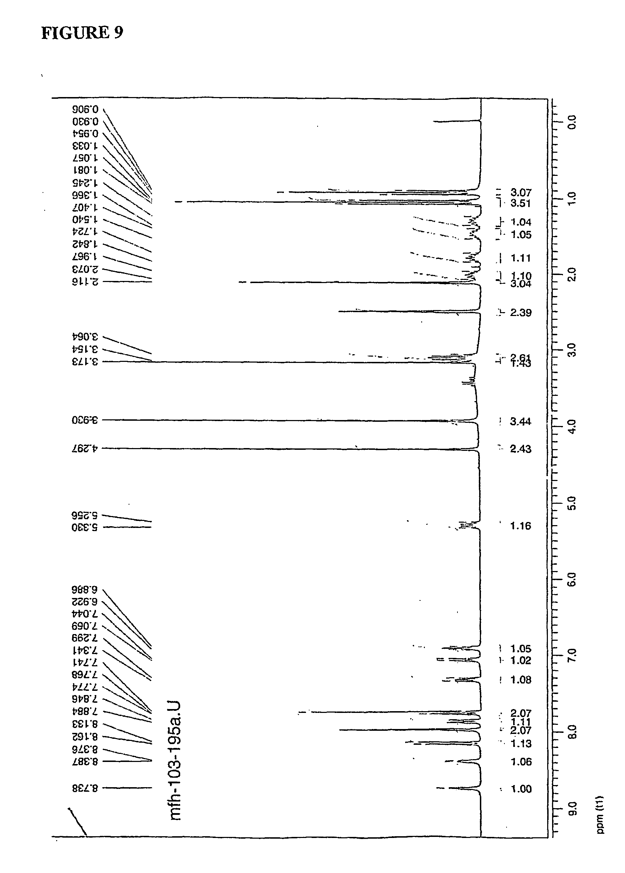

**Figure 9** shows the ¹H-NMR spectrum of a tartaric acid addition salt of the compound of formula I. The 300-MHz ¹H-NMR spectra was obtained at 300 K using 15 mg of the tartaric acid addition salt of N-ethyl-N'-[2-methoxy-4-(5-methyl-4-{[(1S)-1-pyridin-3-ylbutyl]amino}pyrimidin-2-yl)phenyl]urea dissolved in 0.5 mL of DMSO-*d₆*.

### DETAILED DESCRIPTION OF THE INVENTION

In a first aspect, the present invention provides an acid addition salt of a compound of Formula **I**:

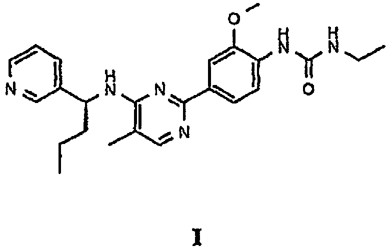

The compound of Formula I is N-ethyl-N'-[2-methoxy-4-(5-methyl-4-{[(1S)-1-pyidin-3-ylbutyl]amino}pyrimidin-2-yl)phenyl]urea,

The acid is selected from the group consisting of citric acid, hydrochloric acid, methanesulfonic acid, oxalic acid and tartaric acid.

In a preferred embodiment, the acid addition salt is a citric acid addition salt.

In another preferred embodiment, the acid addition salt is a hydrochloric acid addition salt. More preferably, the acid addition salt is a dihydrochlozic acid addition salt.

In yet another preferred embodiment, the acid addition salt is a methanesulfonic acid addition salt. More preferably, the acid addition salt is a di-methanesulfonic acid addition salt.

In still another preferred embodiment, the acid addition salt is an oxalic acid addition salt. More preferably, the acid addition salt is a mono-oxalic acid addition salt.

In still another preferred embodiment, the acid addition salt is a tartaric acid addition salt. More preferably, the acid addition salt is a mono-tartaric acid addition salt.

Preferably, the acid addition salt is in a crystalline form.

Acid addition salts of the first aspect are well-suited to pharmaceutical formulation as either solution or solid dosage forms. For example, the solubility in water at 24 ± 3°C for each of the citric acid, hydrochloric acid, methanesulfonic acid, oxalic acid and tartaric acid salts of the compound of Formula I is >2.1 mg/mL. It has been shown that these particular salts are also non-hygroscopic and this remains unchanged when stored in a humid environment. The lower hygroscopicity is a further advantage for processing and storing such acid . addition salts.

Further, acid addition salts of the compound of formula I possess valuable pharmacological properties and may, for example, be used as anti-tumour agents, and as vascular targeting agents for cancer chemotherapy.

Accordingly, in a second aspect, the present invention provides an acid addition salt according to the first aspect for use in a method of treating a hyperproliferation-related disorder.

One pharmacologically important mechanism of inhibiting cellular proliferation is by means of binding tubulin. Tubulin is an asymmetric dimer composed of alpha and beta subunits, that polymerizes to form structural components of the cytoskeleton called microtubules. Microtubules must be highly dynamic in order to carry out many of their functions. At certain stages of the cell cycle, or in particular cell types or organelles, stable miorotubules are required, such as for transport within axons or for ciliary and flagellar movement. Micro-tubules assemble during the G2 phase of the cell cycle, and participate in the formation of the mitotic spindle which facilitates the segregation of sister chromatids during the process of cell division. The essential role of microtubules in cell division and the ability of drugs that interact with tubulin to interfere with the cell cycle have made tubulin a successful target for applications that include anti-cancer drugs, fungicides, and herbicides.

Preferably, the hyperproliferation-related disorder is treatable by the modulation of microtubule polymerisation.

The compound of formula I, and by analogy its acid addition salts, inhibits tubulin polymerisation with an IC50 of 3µM. The compound also displays vascular targeting activity in *in vivo* tumour models. The inhibition of tubulin polymerisation makes a compound of formula I suitable for the treatment of cancer, viral and bacterial infections, vascular restenosis, inflammatory diseases, autoimmune diseases, and psoriasis.

Therefore acid addition salts of the compound of formula I are useful in the treatment of sarcomas, carcinomas and/or leukemias. Exemplary disorders for which the subject method can be used alone or as part of a treatment regimen include: fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, Cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, and retinoblastoma.

Acid addition salts of the compound of formula I are useful in the treatment of diseases such as carcinomas forming from tissue of the breast, prostate, kidney, bladder or colon.

Acid addition salts of the compound of formula I, especially the crystal forms of these acid addition salts, are useful to treat hyperplastic or neoplastic disorders arising in adipose tissue, such as adipose cell tumors, e.g., lipomas, fibrolipomas, lipoblastomas, lipomatosis, hibemomas, hemangiomas and/or liposarcomas.

In still other embodiments, infectious and parasitic agents (e.g. bacteria, trypanosomes, fungi, etc) can also be controlled using the acid addition salts of the first aspect.

The invention relates also to the acid addition salts of the invention for use in the treatment of warm-blooded animals suffering from said diseases, especially a tumour disease, wherein a quantity of an acid addition salt of the compound of formula I which is effective against the disease concerned, especially a quantity with antiproliferative and especially tumour inhibiting efficacy, is administered to warm-blooded animals in need of such treatment. The acid addition salt of the compound of formula I can also be used for the inhibition of tubulin or for the preparation of pharmaceutical compositions for use in treating the human or animal body, especially for the treatment of carcinomas forming from tissue of the breast, prostate, kidney, bladder or colon. Depending on species, age, individual condition, mode of administration, and the clinical picture in question, effective doses, for example daily doses of about 1-2500 mg, preferably 1-1000 mg, especially 5-500 mg, are administered to warm-blooded animals of about 70 kg bodyweight.

In a third aspect, the present invention provides the use of an acid addition salt according to the first aspect in the preparation of a medicament for the treatment of a hyperproliferation-related disorder.

In a fourth aspect, the present invention provides a pharmaceutical composition comprising an acid addition salt according to the first aspect and a pharmaceutically acceptable carrier.

Accordingly, the invention relates also to pharmaceutical preparations which contain an effective amount, especially an effective amount for prevention or treatment of one of the said diseases, of an acid addition salt of the compound of formula I, together with pharmaceutically acceptable carriers which are suitable for topical, enteral, for example oral or rectal, or parenteral administration and may be inorganic or organic and solid or liquid. Especially tablets or gelatin capsules containing the active substance together with diluents, for example lactose, dextrose, sucrose, mannitol, sorbitol, cellulose, and/or glycerin, and/or lubricants, for example silica, talc, stearic acid, or salts thereof, typically magnesium or calcium stearate, and/or polyethylene glycol, are used for oral administration. Tablets may likewise contain binders, for example magnesium aluminium silicate, starches, typically corn, wheat or rice starch, gelatin, methylcellulose, sodium carboxymethylcellulose and/or polyvinylpyrrolidone, and, if so desired, disintegrants, for example starches, agar, alginic acid, or a salt thereof, typically sodium alginate, and/or effervescent mixtures, or adsorbents, colouring agents, flavours, and sweetening agents.

The pharmacologically active compounds of the present invention may further be used in the form of preparations for parenteral administration or infusion solutions. Such solutions are preferably isotonic aqueous solutions or suspensions, these possibly being prepared before use, for example in the case of lyophilised preparations containing the active substance either alone or together with a carrier, for example mannitol. The pharmaceutical substances may be sterilised and/or may contain excipients, for example preservatives, stabilisers, wetting agents and/or emulsifiers, solubilisers, salts for the regulation of osmotic pressure, and/or buffers. The present pharmaceutical preparations which, if so desired, may contain further pharmacologically active substances, such as antibiotics, are prepared in a manner known per se, for example by means of conventional mixing, granulating, coating, dissolving or lyophilising processes, and contain from about 1% to 100%, especially from about 1 % to about 20%, of the active substance or substances.

In order that the nature of the present invention may be more clearly understood, preferred forms thereof will now be described with reference to the following non-limiting examples.

### REFERENCE EXAMPLE 1

### Synthesis of Free Base

The synthesis of N-ethyl-N'-[2-methoxy-4-(5-methyl-4-{[(1S)-1-pyridin-3-ylbutyl]amino}pyrimidin-2-yl)phenyl]urea is described in International Patent Application No. PCT/AU2004/001689 ((WO 2005/054199). The compound can also be prepared in two steps as outlined below:

Mono-addition of 1(*S*)-pyridin-3-yl-butylamine to 2,4-dichloro-5-methylpyrimidine is conducted in a solvent such as N-methylpyrrolidinone at ∼50°C for 15 h. After extractive work-up with a solvent such as toluene, the intermediate is purified by an acid-base cycle and is used in the next step as a solution in, for example, a mixture of toluene and ethanol.

The second step of the synthesis is a Suzuki coupling reaction of the intermediate prepared above with 1-ethyl-3-(2-methoxy-4-(4,4,5,5-tetramethyl-1,3,2-dioxaborolane-2-yl)phenyl]urea. The product is isolated by extraction from the reaction mixture with a solvent such as isopropyl acetate and is purified by acid-base extraction and treatment with a palladium scavenger (such as solid-supported reagent) and isolated by trituration with acetonitrile.

### EXAMPLE 2

### Synthesis of Citric Acid Addition Salt

The citric acid addition salt is prepared from N-ethyl-N'-[2-methoxy-4-(5-methyl-4-{[(1S)-1-pyridin-3-ylbutyl]amino}pyrimidin-2-yl)phenyl]urea by the addition of 0.66 equivalents of a solution of citric acid in methanol to the free base dissolved in methanol. The methanol is removed *in vacuo* and the resulting solid triturated with ether and petroleum spirit.

The crystal form of N-ethyl-N'-[2-methoxy-4-(5-methyl-4-{[(1S)-1-pyridin-3-ylbutyl]amino}pyrimidin-2-yl)phenyl]urea citrate salt is characterised as a pale yellow solid and is not hygroscopic. The structure was confirmed by ¹H- and ¹³C- nuclear magnetic resonance spectroscopy and mass spectrometry.

¹H-n.m.r. (DMSO-d6) δ0.93 (t, 3H, *J* = 7.2 Hz), 1.06 (t, 3H, *J* = 7.2 Hz), 1.23-1.37 (m, 1H), 1.41-1.54 (m, 1H), 1.73-1.84 (m, 1H), 1.96-2.08 (m, 1H), 2.12 (s, 3H), 2.64 and 2.74 (AB quartet, 2.6H, *J* = 15.3Hz), 3.07-3.18 (m, 2H), 3.93 (s, 3H), 5.26-5.34 (m, 1H), 6.91 (t, 1H, *J* = 5.4 Hz), 7.10 (d, 1H, *J* = 7.2 Hz), 7.32 (ddd, 1H, *J* = 7.8,4.8,0.6 Hz), 7.74-7.77 (m, 2H), 7.85-7.89 (m, 1H), 7.97-7.98 (m, 2H) 8.15 (d, 1H, *J* = 8.4 Hz), 8.38 (dd, 1H, *J* = 4.8, 1.8 Hz), 8.73 (d, 1H, *J* = 1.8 Hz).

¹³C-n.m.r. (DMSO-d6) δ13.5, 13.6, 15.1, 19.3, 33.8, 37.9, 42.7, 52.1, 55.5, 72.2, 108.9, 111.4, 116.7, 120.0, 123.3, 130.4, 131.3, 133.7, 140.1, 146.6, 147.6, 148.4, 152.9, 154.7, 159.8, 160.1, 171.1, 174.6.

### EXAMPLE 3

### Synthesis of Dihydrochloride Acid Addition Salt

The hydrochloric acid addition salt is prepared from the material prepared as described above through the addition of excess ethanolic hydrogen chloride and subsequent crystallization with a solvent such as methyl acetate.

The crystal form of N-ethyl-N'-[2-methoxy-4-(5-methyl-4-{[(1S)-1-pyridin-3-ylbutyl]amino}pyrimidin-2-yl)phenyl]urea dihydrochloride salt is characterised as a pale yellow solid and is not hygroscopic (m.p. 241 - 248°C). The structure was confirmed by ¹H- and ¹³C- nuclear magnetic resonance spectroscopy, mass spectrometry, IR and UV spectroscopy as well as elemental analysis and X-ray powder diffraction analysis.

Figures 1 to 3 depict respectively the X-ray powder diffraction spectra, the ¹H-NMR spectrum and the ¹³C-NMR spectrum of the dihydrochloride salt.

¹H-n.m.r. (d6-DMSO) δ 0.92 (t, 3H, *J* = 7.5 Hz), 1.04 (t, 3H, *J* = 7.2 Hz), 1.13-1.38 (m, 1H), 1.44-1.55 (m, 1H), 1.83-1.94 (m, 1H), 2.08-2.21 (m, 1H), 2.27 (s, 3H), 3.08-3.12 (m, 2H), 3.97 (s, 3H), 5.63-5.66 (m, 1H), 7.20 (t, 1H, *J* = 5.4 Hz), 7.85-7.92 (m, 2H), 8.12 (s, 1H), 8.34 (d, 1H, *J* = 0.9 Hz), 8.36 (s, 1H), 8.64 (d, 1H, *J* = 8.4 Hz), 8.71 (m, 1H), 9.09 (m, 1H), 9.10 (m, 1H).

¹³C-n.m.r. (d6-DMSO) δ 13.42,13.66, 15.02, 19.11, 33.74, 36.65, 52.79, 56.44, 109.70, 113.67, 116.48, 121.29, 122.15, 126.98, 134.78, 140.19, 140.57, 142.76, 143.85, 146.74, 154.45, 155.00, 161.27

### EXAMPLE 4

### Synthesis of Di-methanesulfonic Acid Addition Salt

The di-methanesulfonic acid addition salt is prepared from N-ethyl-N'-[2-methoxy-4-(5-methyl-4-{[(1S)-1-pyridin-3-ylbutyl]amino}pyrimidin-2-yl)phenyl]urea by the addition of 2.0 equivalents of a solution of methanesulfonic acid in tetrahydrofuran to the free base dissolved in methanol. The methanol is removed *in vacuo* and the resulting solid triturated with ether and petroleum spirit.

The crystal form of N-ethyl-N'-[2-methoxy-4-(5-methyl-4-{[(1S)-1-pyridin-3-ylbutyl]amino}pyrimidin-2-yl)phenyl]urea di-methanesulfonate salt is characterised as a yellow solid and is not hygroscopic. The structure was confirmed by ¹H- and ¹³C- nuclear magnetic resonance spectroscopy and mass spectrometry.

¹H-n.m.r. (DMSO-d6) δ0.96 (t, *J* = 7.2 Hz, 3H), 1.07 (t, *J* = 7.2 Hz, 3H), 1.27-1.41 (m, 1H), 1.49-1.61 (m, 1H), 1.87-1.99 (m, 1H), 2.11-2.24 (m, 1H), 2.29 (s, 3H), 2.43 (s, 6H), 3.12 (q, *J* = 7.2 Hz, 2H), 3.97 (s, 3H), 5.67-5.75 (m, 1H), 7.13 (br s, 1H), 7.66 (d, *J* = 1.8 Hz, 1H), 7.79 (dd, *J* = 8.7, 1.8 Hz, 1H), 8.10 (dd, *J* = 8.1, 5.7 Hz, 1H), 8.19 (d, *J* = 0.9 Hz, 1H), 8.34 (s, 1H), 8.38 (d, *J* = 8.7 Hz, 1H), 8.79 (d, *J* = 8.4 Hz, 1H), 8.85 (d, *J* = 5.4 Hz, 1H), 8.97 (d, *J* 7.5 Hz, 1H), 9.14 (s, 1H).

### EXAMPLE 5

### Synthesis of Oxalic Acid Addition Salt

The oxalic acid addition salt is prepared from N-ethyl-N'-[2-methoxy-4-(5-methyl-4-{[(1S)-1-pyridin-3-ylbutyl]amino}pyrimidin-2-yl)phenyl]urea by the addition of 0.5 equivalents of a solution of oxalic acid in methanol to the free base dissolved in methanol. The methanol is removed *in vacuo* and the resulting solid triturated with ether and petroleum spirit.

The crystal form of N-ethyl-N'-[2-methoxy-4-(5-methyl-4-{[(1S)-1-pyridin-3-ylbutyl]amino}pyrimidin-2-yl)phenyl]urea mono-oxalate salt is characterised as a pale yellow solid and is not hygroscopic. The structure was confirmed by ¹H- and ¹³C- nuclear magnetic resonance spectroscopy and mass spectrometry.

¹H-n.m.r. (DMSO-d6) δ0.93 (t, 3H, *J* = 7.2 Hz), 1.06 (t, 3H, *J* = 7.2 Hz), 1.25-1.37 (m, 1H), 1.41-1.54 (m, 1H), 1.74-1.86 (m, 1H), 1.98-2.07 (m, 1H), 2.13 (d, 3H, *J* = 0.6 Hz), 3.07-3.14 (m, 2H), 3.94 (s, 3H), 5.29-5.36 (m, 1H), 6.93 (t, 1H, *J* = 5.4 Hz), 7.32-7.36 (m, 2H), 7.72-7.77 (m, 2H), 7.86-7.90 (m, 1H), 8.00 (d, 1H, *J* = 0.9 Hz), 8.03 (s, 1H), 8.18 (d, 1H, *J* = 8.4 Hz), 8.39 (dd, 1H, *J* = 4.8, 1.5 Hz), 8.74 (d, 1H, *J* = 1.8 Hz).

¹³C-n.m.r. (DMSO-d6) δ13.4, 13.5, 15.2, 19.3, 33.7, 37.7, 52.3, 55.6, 108.9, 111.6, 117.0, 120.2, 123.4, 129.1, 131.8, 133.9, 139.9, 146.6, 147.6, 148.3, 151.2, 154.6, 159.4, 160.0, 161.6.

### EXAMPLE 6

### Synthesis of Tartaric Acid Addition Salt

The tartaric acid addition salt is prepared from N-ethyl-N'-[2-methoxy-4-(5-methyl-4-{[(1S)-1-pyridin-3-ylbutyl]amino}pyrimidin-2-yl)phenyl]urea by the addition of 0.5 equivalents of a solution of tartaric acid in methanol to the free base dissolved in methanol. The methanol is removed *in vacuo* and the resulting solid triturated with ether and petroleum spirit.

The crystal form of N-ethyl-N'-[2-methoxy-4-(5-methyl-4-{[(1S)-1-pyridin-3-ylbutyl]amino}pylimidin-2-yl)phenyl]urea tartrate salt is characterised as a pale yellow solid and is not hygroscopic. The structure was confirmed by ¹H- and ¹³C- nuclear magnetic resonance spectroscopy and mass spectrometry.

1H-n.m.r. (DMSO-d6) δ0.93 (t, 3H, *J* = 7.2 Hz), 1.06 (t, 3H, *J* = 7.2 Hz), 1.25-1.37 (m, 1H), 1.41-1.54 (m, 1H), 1.72-1.84 (m, 1H), 1.97-2.07 (m, 1H), 2.12 (s, 3H), 3.06-3.15 (m, 2H), 3.93 (s, 3H), 4.30 (s, 2H), 5.26-5.33 (m, 1H), 6.90 (t, 1H, *J* = 5.4 Hz), 7.06 (d, 1H, *J* = 7.5 Hz), 7.32 (dd, 1H, *J* = 7.8, 4.8 Hz), 7.74-7.77 (m, 2H), 7.85-7.88 (m, 1H), 7.97 (br s, 2H), 8.15 (d, 1H, *J* = 8.7 Hz), 8.38 (d, 1H, *J* = 3.3 Hz), 8.74 (br s, 1H).

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

## Claims

1. An acid addition salt of a compound of Formula I: 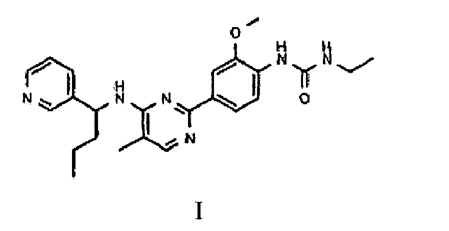 wherein the acid is selected from the group consisting of citric acid, hydrochloric acid, methanesulfonic acid, oxalic acid and tartaric acid.

2. An acid addition salt according to claim 1, wherein the salt is a citric acid addition salt.

3. An acid addition salt according to claim 1, wherein the salt is a hydrochloric acid addition salt.

4. An acid addition salt according to claim 1, wherein the salt is a methanesulfonic acid addition salt.

5. An acid addition salt according to claim 1, wherein the salt is an oxalic acid addition salt.

6. An acid addition salt according to claim 1, wherein the salt is a tartartic acid addition salt.

7. An acid addition salt according to any one of claims 1 to 6, wherein the salt is in a crystalline form.

8. An acid addition salt according to any one of claims 1 to 7 for use in the treatment of a hyperproliferation-related disorder.

9. An acid addition salt according to claim 8, wherein the hyperproliferation-related disorder is treatable by the modulation of microtubule polymerisation.

10. An acid addition salt according to claim 8 or claim 9, wherein the hyperproliferation-related disorder is selected from the group including cancer, viral and bacterial infections, vascular restenosis, inflammatory diseases, autoimmune diseases and psoriasis.

11. Use of an acid addition salt according to any one of claims 1 to 7, in the preparation of a medicament for the treatment of a hyperproliferation-related disorder as defined in one of claims 8 to 10.

12. A pharmaceutical composition comprising:
an acid addition salt according to any one of claims 1 to 7; and
a pharmaceutically acceptable carrier.

## Patentansprüche

1. Säureadditionssalz einer Verbindung der Formel I: 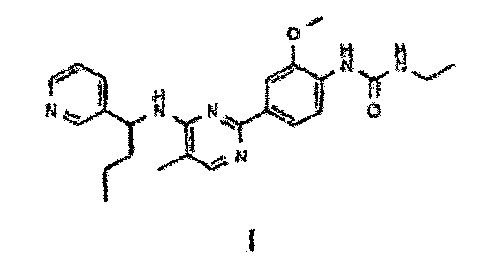 worin die Säure aus der aus Citronensäure, Salzsäure, Methansulfonsäure, Oxalsäure und Weinsäure bestehenden Gruppe ausgewählt ist.

2. Säureadditionssalz nach Anspruch 1, worin das Salz ein Citronensäureadditionssalz ist.

3. Säureadditionssalz nach Anspruch 1, worin das Salz ein Salzsäureadditionssalz ist.

4. Säureadditionssalz nach Anspruch 1, worin das Salz ein Methansulfonsäureadditionssalz ist.

5. Säureadditionssalz nach Anspruch 1, worin das Salz ein Oxalsäureadditionssalz ist.

6. Säureadditionssalz nach Anspruch 1, worin das Salz ein Weinsäureadditionssalz ist.

7. Säureadditionssalz nach einem der Ansprüche 1 bis 6, worin das Salz in kristalliner Form vorliegt.

8. Säureadditionssalz nach einem der Ansprüche 1 bis 7 zur Verwendung bei der Behandlung einer hyperproliferationsbedingten Störung.

9. Säureadditionssalz nach Anspruch 8, worin die hyperproliferationsbedingte Störung durch die Modulierung von Mikrotubulipolymerisation behandelbar ist.

10. Säureadditionssalz nach Anspruch 8 oder Anspruch 9, worin die hyperproliferationsbedingte Störung aus der aus Krebs, Viren- und Bakterieninfektionen, Gefäßrestenose, Entzündungskrankheiten, Autoimmunerkrankungen und Psoriasis bestehenden Gruppe ausgewählt ist.

11. Verwendung eines Säureadditionssalzes nach einem der Ansprüche 1 bis 7 bei der Herstellung eines Medikaments zur Behandlung einer hyperproliferationsbedingten Störung wie in einem der Ansprüche 8 bis 10 definiert.

12. Pharmazeutische Zusammensetzung, die Folgendes umfasst:
ein Säureadditionssalz nach einem der Ansprüche 1 bis 7; und
einen pharmazeutisch annehmbaren Träger.

## Revendications

1. Sel d'addition d'acide d'un composé de formule I : 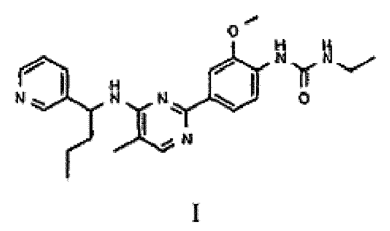 dans lequel l'acide est choisi dans le groupe constitué par l'acide citrique, l'acide chlorhydrique, l'acide méthanesulfonique, l'acide oxalique et l'acide tartrique.

2. Sel d'addition d'acide selon la revendication 1, dans lequel le sel est un sel d'addition d'acide citrique.

3. Sel d'addition d'acide selon la revendication 1, dans lequel le sel est un sel d'addition d'acide chlorhydrique.

4. Sel d'addition d'acide selon la revendication 1, dans lequel le sel est un sel d'addition d'acide méthanesulfonique.

5. Sel d'addition d'acide selon la revendication 1, dans lequel le sel est un sel d'addition d'acide oxalique.

6. Sel d'addition d'acide selon la revendication 1, dans lequel le sel est un sel d'addition d'acide tartrique.

7. Sel d'addition d'acide selon l'une quelconque des revendications 1 à 6, dans lequel le sel est sous une forme cristalline.

8. Sel d'addition d'acide selon l'une quelconque des revendications 1 à 7, pour une utilisation dans le traitement d'un trouble lié à l'hyperprolifération.

9. Sel d'addition d'acide selon la revendication 8, dans lequel le trouble lié à l'hyperprolifération peut être traité par modulation de la polymérisation de microtubules.

10. Sel d'addition d'acide selon la revendication 8 ou la revendication 9, dans lequel le trouble lié à l'hyperprolifération est choisi dans le groupe comprenant le cancer, les infections virales et bactériennes, la resténose vasculaire, les maladies inflammatoires, les maladies auto-immunes et le psoriasis.

11. Utilisation d'un sel d'addition d'acide selon l'une quelconque des revendications 1 à 7, dans la préparation d'un médicament destiné au traitement d'un trouble lié à l'hyperprolifération tel que défini selon l'une quelconque des revendications 8 à 10.

12. Composition pharmaceutique comprenant .
un sel d'addition d'acide selon l'une quelconque des revendications 1 à 7 ; et
un véhicule pharmaceutiquement acceptable.
